# EUROPEAN PATENT SPECIFICATION

(11) **EP 3 437 638 B1**
(45) Date of publication and mention of the grant of the patent: **18.11.2020**
(21) Application number: 18186559.3
(22) Date of filing: 31.07.2018
(51) Int. Cl.: A61K 31/201, A61K 31/198, A61K 31/065, A61P 25/02

(54) **NERVONIC ACID, CURCUMIN AND A BASIC AMINOACID FOR THE TREATMENT OF PATHOLOGIES ASSOCIATED WITH PERIPHERAL NERVES TROPHISM ALTERATIONS**
NERVONSÄURE, CURCUMIN UND EINE BASICHE AMINOSÄURE ZUR BEHANDLUNG VON MIT PERIPHEREN NERVEN TROPHISM ÄNDERUNGEN VERBUNDENEN KRANKHEITEN
ACID NERVONIQUE, CURCUMIN ET UN AMINOACIDE BASIQUE POUR TRAITER LES PATHOLOGIES ASSOCIÉES AVEC DES ALTERATIONS DU TROPHISME DES NERFS PERIPHERIQUES

(30) Priority: 01.08.2017 IT 201700088621
(43) Date of publication of application: 06.02.2019
(73) Proprietor: Inpha Research S.r.l., 20122 Milano MI (IT)
(72) Inventor: Castelli, Simone, 23900 Lecco LC (IT); Samaritani, Giuseppe, 23900 Lecco LC (IT)
(74) Representative: Perani & Partners S.p.A.

(56) References cited:
- WO-A2-2004/047717
- DATABASE WPI Week 200982 Thomson Scientific, London, GB; AN 2009-R75712 XP002779889, & CN 101 579 334 A (LI Z) 18 November 2009 (2009-11-18)
- DATABASE WPI Week 201641 Thomson Scientific, London, GB; AN 2016-318946 XP002779890, & JP 2016 088857 A (NIMURA Y) 23 May 2016 (2016-05-23)
- KUMAR A ET AL: "Possible nitric oxide modulation in protective effect of (Curcuma longa, Zingiberaceae) against sleep deprivation-induced behavioral alterations and oxidative damage in mice", PHYTOMEDICINE, ELSEVIER, AMSTERDAM, NL, vol. 15, no. 8, 1 August 2008 (2008-08-01) , pages 577-586, XP022819241, ISSN: 0944-7113, DOI: 10.1016/J.PHYMED.2008.02.003 [retrieved on 2008-06-30]
- PATZKÓ AGNES ET AL: "Curcumin derivatives promote Schwann cell differentiation and improve neuropathy in R98C CMT1B mice.", BRAIN : A JOURNAL OF NEUROLOGY DEC 2012, vol. 135, no. Pt 12, December 2012 (2012-12), pages 3551-3566, XP002738484, ISSN: 1460-2156
- LAUGHLIN: PNAS, JANUARY 21.2003, VOL. 100 N° 2, 715-720, 2003,
- GARNIER: THE JOURNAL OF NEUROSCIENCE, SEPTEMBER 3, 2003, 23(22):7967-7973, 2003,

## Description

### FIELD OF THE INVENTION

The present invention relates to the association of nervonic acid and curcumin for use in the treatment of pathologies associated with the alteration of the trophism of the peripheral nerves and related oral compositions, in particular in the form of food supplements.

### STATE OF THE ART

Nervonic acid is a ω9 fatty acid, also known as selacholeic acid or cis-15tetracosenoic acid, a monounsaturated analogue of lignoceric acid. It exists naturally as an elongation product of oleic acid: its immediate precursor is erucic acid.

In living organisms, it is present at the level of the white matter and in the peripheral nervous tissue, in particular at the level of the myelin sheath of nerve fibres (1). In fact, nervonic acid is a cerebroside (subcategory of sphingolipids). It normally represents about 40% of the total sphingolipids (1).

From a physiological point of view, nervonic acid is a regulator of the Ca²⁺ ion channel in the cellular membrane of nervous tissues, thus playing an important role in the control of calcium levels of cytosol. It is an essential component for the growth and the good development of the brain and is in fact contained in breast milk. In this regard, some studies have shown that nervonic acid is involved as an intermediate in the biosynthesis of myelin of nerve cells (2, 4).

As a consequence of its biochemical characteristics, nervonic acid has shown the ability to improve brain functions and prevent demyelination (3). Some interesting evidences have turned out in particular in the treatment of genetic disorders of lipid metabolism, such as Zellweger syndrome or adrenoleukodystrophy (2, 6).

In particular, demineralization in adrenoleukodystrophy (ALD) is associated with an accumulation of very long saturated fatty acids resulting from a genetic defect of the peroxisomal beta-oxidation system responsible for the increase of the chain of such fatty acids. Beta-oxidation of nervonic acid occurs in peroxisomes and this oxidation is compromised in patients with X-ALD (2). Because of different mutations, people with these disorders have ineffective peroxisomes (4) (6). As a further evidence of the importance of nervonic acid for the proper functioning of the nervous system, it has been observed that the brain of patients with ALD shows reduced levels of nervonic acid, 24:1 (n-9) and increased levels of stearic acid, 18:0.

The increased production of ROS (Reactive Oxygen Species) and a reduced scavenging action of free radicals by antioxidant substances physiologically present in the body is a common situation in individuals suffering from peripheral neuropathies. Thanks to its antioxidant, detoxifying, anti-inflammatory and neuroprotective properties, nervonic acid acts on several fronts, contributing both to the rebalancing of the redox mechanisms of the cells and, more generally, to the improvement of neuropathic pain.

Turmeric is a perennial herbaceous plant of the Zinziberaceae family, characterized by the presence of an underground rhizome that can reach a size of 10 cm in diameter. A native of South Asia, in Java, it is grown in various tropical areas in Central America, Antilles and Malaysia. The rhizome phytocomplex consists mainly of two groups of molecules called curcuminoids, among which curcumin is the most represented and active, and triterpenes, constitutive of essential oil (most representative ones: zingiberene, borneol, alpha-phellandrene, turmerone). The main activity attributed to the phytocomplex is the anti-inflammatory one. In particular, curcumin has been tested on animal models of acute, chronic and subacute inflammation. Orally it has shown to be equal to phenylbutazone and cortisone in acute models, but only equal to half of phenylbutazone in chronic models. Curcumin is more effective than ibuprofen in stabilizing hepatic liposomal membranes and reduces hepatic oxidative phosphorylation. In a double-blind study of post-operative inflammation, curcumin was more effective than phenylbutazone. One possible mechanism responsible for the anti-inflammatory activity is the dual inhibition of 5-LOX and COX. In fact, curcumin strongly inhibits the formation of leukotrienes and weakly inhibits the formation of prostaglandins. In peripheral neuropathies related to diabetes, curcumin has proved effective in decreasing the state of inflammation and pain on an animal model. The hypothesized mechanisms of action are:
- Inhibition of TNF-alpha and nitric oxide production
- Decreased levels of nitrites in the brain
- Inhibition of the production of interleukin-8 (IL-8), interleukin-1β (IL-Iβ) (7), (8). Document WO2004047717 discloses the activity of curcumin in treating topically peripheral neuropathies associated with diabetes.

### SUMMARY OF THE INVENTION

The applicant has now found that the association of nervonic acid and curcumin with an aminoacid containing at least an amino group besides the aminoacid amino group can be advantageously employed in the treatment of alterations in the trophism of the nerves.

Therefore, the object of the present invention is the aforementioned association of active ingredients for use in the treatment of pathologies associated with the aforementioned alteration.

Further object are oral compositions in unitary dosage form comprising as active ingredient an association of nervonic acid and curcumin with an aminoacid containing at least an amino group besides the aminoacid amino group in combination with suitable excipients and/or diluents. The invention is defined by the claims.

### DETAILED DESCRIPTION OF THE INVENTION

For the purposes of the present invention, unitary or single oral compositions mean oral formulations, which can be taken in a single dose, such as e.g. swallowable and water-dispersible tablets, rigid or soft (soft gel) capsules of animal or vegetable gelatine, powders or granules, in form of single-dose sachets, for extemporaneous aqueous dispersion.

For the purposes of the present invention, the expression "*comprise*/*contain n components"* does not exclude the presence of further components in addition to the n components explicitly reported. For the purposes of the present invention, the expression in which a subject *"consists or is formed or composed of n components"* means that the subject excludes the presence of further components besides the n components already listed.

The pathologies associated with alterations in the trophism of the peripheral nerves treated with the association according to the present invention are neural complications of diabetes, neuritis on an inflammatory basis, neuropathic pain syndromes on a traumatic or rheumatologic basis.

Nervonic acid, as specified above, is an omega-9 fatty acid, therefore it has a poor solubility in water. This feature makes it difficult to be conveyed in pharmaceutical forms such as water-soluble powders.

Furthermore, due to its nature of fatty acid, its bioavailability is closely linked to the biliary secretion of the individual that, especially in the elderly, may be compromised or reduced due to hepatobiliary pathologies or reduced consumption of foods on a fat basis.

The applicant has also found that it is possible to increase the bioavailability and the solubility in water by conveying it with a basic amino acid, in free base form.

A basic amino acid in free base form means in particular an amino acid that contains at least 2 amino groups, i.e. 1 free amino group more than the amino in the amino acid group, in which the basic amino acid is present in free base form, and therefore the second amino group is not in salified form.

Examples of such basic amino acids are for example lysine, ornithine or arginine.

In particular, L-arginine in the form of an unsupplied free base contains the guanidyl group (with 3 nitrogen atoms more) in addition to the typical amino in the amino acid group.

The introduction of L-arginine in free base form in the aforesaid composition is able to favour the salification of the nervous acid for which the carboxylic function is responsible, thus determining its concomitant dispersion in water and dissolution in enteric secretions. In fact, the combination of nervonic acid and L-arginine gives the formation of arginine nervonate, an amphiphilic molecule that acts as a surfactant. Thanks to the formation of this molecule, due to its surfactant structure, not only will the solubility of the nervonic acid be improved in water, making it possible to incorporate the nervonic acid in the form of powders or water-dispersible tablets, but it will allow delineating a possible increase in the bioavailability of nervonic acid and plausibly of other components of the formulation by exploiting the ability of L-arginine to act as a salifying agent. In particular, given the presence in the aforesaid composition of curcumin in the form of Turmeric root extract, which is known for its poor oral bioavailability in humans due to poor solubility in water, the presence of L-arginine may also promote the dispersion in water of curcumin with the same mechanism of polyphenolic substances and flavonoids which, as already known, are soluble in water in alkaline conditions.

Preferably, the oral compositions in the form of unit doses object of the present invention comprise between 50 and 500 mg of nervonic acid, between 50 and 500 mg of Turmeric dried root extract titrated in curcumin.

This composition will preferably also comprise the free base arginine in molar ratios with respect to the nervonic acid ≥ 1.

More preferably, L-arginine is present in the oral compositions according to the present invention in amounts such that the relative extemporaneous aqueous dispersion has an internal pH range of 7.5 to 8.5.

For the purposes of the present invention, internal interval between 7.5 and 8.5 means an interval whose extremes (7.5 and 8.5) are not included, in other words a neighbourhood that can be mathematically expressed as 7.5 < pH > 8.5.

In this pH neighbourhood, in fact, there is certainty that most of the nervonic acid and curcumin will be in a water-dispersible form. This pH range is obtained when the weight ratio between nervonic acid in free carboxylic form and arginine in free form is between 1:1.2 and 1:2.4.

According to a preferred solution, the oral compositions object of the present invention contain a vitamin B complex and folic acid.

According to a more preferred solution, said vitamin complex B consists of at least one of the vitamins B1, B2, B3, B5, B6 and B12.

According to an even more preferred solution, in the oral compositions according to the present invention said vitamins B1, B2, B3, B5, B6 are each contained in amounts ranging from 1 to 50 mg, while each of said vitamin B12 and folic acid is contained in amounts ranging from 1 to 1000 µg.

An example of an oral composition comprising nervonic acid, Turmeric, vitamins B1, B6 and B12 and arginine is given for illustrative, but not limitative purposes. The oral compositions in unitary dosage form are in particular in the form of food supplements, or alternatively the supplements comprise said oral compositions.

For the purposes of the present invention, food supplements are defined according to the definition given in Article 2 of Legislative Decree No. 169 of 21 May 2004, i.e. food products intended to supplement the common diet and constituting a concentrated source of nutrients, such as vitamins and minerals, or other substances having a nutritional or physiological effect, in particular, but not exclusively, amino acids, essential fatty acids, fibres and extracts of plant origin, both mono-composed and multi-composed, in pre-dosed forms.

### Example of composition

| Component | Amount | VNR¹ % |
|---|---|---|
| Nervonic acid | 250 mg | - |
| Turmeric (Curcuma longa L) dried rhizome extract 95% titrated in curcuminoids | 100 mg | |
| Vitamin B1 | 1.1 mg | 100% |
| Vitamin B6 | 1.4 mg | 100% |
| Vitamin B12 | 2.5 µg | 100% |
| Arginine | 500 mg | - |

| | | |
|---|---|---|
| ¹ reference nutritional values | | |

### Bibliography

1) Rajat Sandhir, Localization of nervonic acid b-oxidation in human and rodent peroxisomes: impaired oxidation in Zellweger syndrome and X-linked adrenoleukodystrophy, Journal of Lipid Research Volume 39, 1998;
2) Bourre JM, Daudu O, Baumann N. Nervonic acid biosynthesis by erucyl-CoA elongation in normal and quaking mouse brain microsomes. Elongation of other unsaturated fatty acyl-CoAs (mono and poly-unsaturated). Biochim. Biophys. Acta. 1976 Jan 22; 424(1):1-7;
3) Sargent, J. R.; Coupland, K.; Wilson, R. "Nervonic acid and demyelinating disease" Medical Hypotheses. 42(4): 237-242. (1994-04-01);
4) Marillia, "Production of Nervonic acid in Brassica carinata for Industrial and Health Applications", Canadian counsil, April 2014. Retrieved 15 October 2015;
5) Martinez M., Mougan I., Fatty acid composition of human brain phospholipids during normal development. J Neurochem.1998;
6) Todd E. Fox, Circulating sphingolipid biomarkers in models of type 1 diabetes, Journal of Lipid Research Volume 52, 2011;
7) Shah BH, Nawaz Z, Pertani SA et al. Inhibitory effect of curcumin, a food spice from turmeric, on platelet-activating factor- and arachidonic acid-mediated platelet aggregation through inhibition of thromboxane formation and Ca 2+ signaling. Biochem Pharmacol 1999; 58:1167-1172;
8) Sharma RA, McLelland HR, Hill KA et al. Pharmacodynamic and pharmacokinetic study of oral Curcuma extract in patients with colorectal cancer. Clin Cancer Res 2001; 7:1894-1900.

## Claims

1. Association of nervonic acid and curcumin for use in the treatment of pathologies associated with the alteration of the trophism of the peripheral nerves, wherein the pathologies are selected from neural complications of diabetes, neuritis on an inflammatory basis, neuropathic pain syndromes on a traumatic or rheumatologic basis, wherein said association is contained in an oral composition in a unitary dosage form containing said association as the sole active ingredient combined with suitable excipients and/or diluents further comprising an amino acid containing at least one amino group besides the amino acid amino group, wherein such basic amino acid is present in free base form.

2. The association for the use according to claim 1, wherein said amino acid containing at least 1 amino group besides the amino acid amino group, wherein such basic amino acid is present in free base form, selected from lysine, ornithine and arginine, preferably it is L-arginine.

3. The association for the use according to claim 2, wherein said amino acid containing at least one amino group besides the amino acid amino group is L-arginine.

4. The association for the use according to anyone of claims 1-3, wherein said oral compositions in a unitary dosage form comprise between 50 and 500 mg of nervonic acid, between 50 and 500 mg of Turmeric dried root extract, titrated in curcumin.

5. The association for the use according to claim 3, wherein the L-arginine in free base form is present in molar ratios ≥ 1 with respect to nervonic acid.

6. The association for the use according to claim 3 or 5 wherein L-arginine is present in said oral compositions in a unitary dosage form in such amounts that the related extemporaneous aqueous dispersion has an internal pH range between 7.5 and 8.5.

7. The association for the use according to anyone of claims 3-6, wherein the weight ratio of nervonic acid: L-arginine in free base form ranges from 1:1.2 to 1:2.4.

8. The association for the use according to anyone of claims 1 - 7, wherein said oral compositions in a unitary dosage form comprise a vitamin B complex and folic acid.

9. The association for the use according to claim 8, wherein said vitamin B complex consists of at least one of the vitamins B1, B2, B3, B5, B6, B12.

10. The association for the use according to claim 9, wherein said vitamins B1, B2, B3, B5, B6 are each contained in amounts ranging from 1 to 50 mg, while each of said B12 and folic acid is contained in amounts ranging from 1 to 1000 µg.

11. Association for the use according to anyone of claims 1-10, wherein said oral compositions are in form of swallowable and water-dispersible tablets, rigid or soft (soft gel) capsules of animal or vegetable gelatine, powders or granules, in form of single-dose sachets, for extemporaneous aqueous dispersion.

12. Association for the use according to anyone of claims 1-11 wherein said oral compositions in unitary dosage form are dietary supplements.

## Patentansprüche

1. Assoziation von Nervonsäure und Curcumin zur Verwendung bei der Behandlung von Pathologien, die mit der Veränderung der Trophik der peripheren Nerven assoziiert sind, wobei die Pathologien aus neuronalen Komplikationen von Diabetes, Neuritis auf entzündlicher Basis, neuropathischen Schmerzsyndromen auf traumatischer oder rheumatologischer Basis ausgewählt sind, wobei die Assoziation in einer oralen Zusammensetzung in Form einer einheitlichen Dosierung enthalten ist, die die Assoziation als den einzigen aktiven Bestandteil kombiniert mit geeigneten Arzneiträgerstoffen und/oder Verdünnungsmitteln enthält, ferner umfassend eine Aminosäure, die mindestens eine Aminogruppe neben der Aminosäuregruppe enthält, wobei eine solche basische Aminosäure in Form einer freien Base vorliegt.

2. Assoziation zur Verwendung nach Anspruch 1, wobei die Ammonsäure, die neben der Aminosäureaminogruppe mindestens 1 Aminogruppe enthält, wobei diese basische Aminosäure in Form einer freien Base vorliegt, die aus Lysin, Ornithin und Arginin, vorzugsweise L-Arginin, ausgewählt ist.

3. Assoziation zur Verwendung nach Anspruch 2, wobei die Ammonsäure, die neben der Aminosäure-Aminogruppe mindestens eine Aminogruppe enthält, L-Arginin ist.

4. Assoziation zur Verwendung nach einem der Ansprüche 1-3, wobei die oralen Zusammensetzungen in Form einer einheitlichen Dosierung zwischen 50 und 500 mg Nervonsäure, zwischen 50 und 500 mg titrierten in Curcumin Kurkuma getrockneten Wurzelextrakt umfassen.

5. Assoziation zur Verwendung nach Anspruch 3, wobei das L-Arginin in Form einer freien Base in Molverhältnis ≥ 1 in Bezug auf Nervonsäure vorliegt.

6. Assoziation zur Verwendung nach Anspruch 3 oder 5, wobei L-Arginin in den oralen Zusammensetzungen in Form einer einheitlichen Dosierung in solchen Mengen vorliegt, dass die entsprechende extemporane wässrige Dispersion einen inneren pH-Bereich zwischen 7,5 und 8,5 aufweist.

7. Assoziation zur Verwendung nach einem der Ansprüche 3-6, wobei das Gewichtsverhältnis von Nervonsäure:L-Arginin in Form einer freien Base im Bereich von 1:1,2 bis 1:2,4 liegt.

8. Assoziation zur Verwendung nach einem der Ansprüche 1-7, wobei die oralen Zusammensetzungen in Form einer einheitlichen Dosierung einen Vitamin-B-Komplex und Folsäure umfassen.

9. Assoziation zur Verwendung nach Anspruch 8, wobei der Vitamin-B-Komplex aus mindestens einem der Vitamine B1, B2, B3, B5, B6, B12 besteht.

10. Assoziation zur Verwendung nach Anspruch 9, wobei die Vitamine B1, B2, B3, B5, B6 jeweils in Mengen im Bereich von 1 bis 50 mg enthalten sind, während B12 und Folsäure jeweils in Mengen im Bereich von 1 bis 1000 µg enthalten sind.

11. Assoziation zur Verwendung nach einem der Ansprüche 1-10, wobei die oralen Zusammensetzungen in Form von schluckbaren und in Wasser dispergierbaren Tabletten, starren oder weichen (soft gel) Kapseln aus tierischer oder pflanzlicher Gelatine, Pulvern oder Granulaten, in Form von Beutelchen für Maßeinheit für eine extemporane wässrige Dispersion vorliegen.

12. Assoziation zur Verwendung nach einem der Ansprüche 1-11, wobei die oralen Zusammensetzungen in Form einer einheitlicher Dosierung Nahrungsergänzungsmittel sind.

## Revendications

1. Association d'acide nervonique et de curcumine destinée à être utilisée dans le traitement de pathologies associées à l'altération du trophisme des nerfs périphériques, où les pathologies sont choisies parmi les complications neuronales du diabète, la névrite sur une base inflammatoire, les syndromes de douleur neuropathique sur une base traumatique ou rhumatologique, où ladite association est contenue dans une composition orale dans une dose unitaire à partir du fait de contenir ladite association en tant que seul ingrédient actif combiné avec des excipients et/ou diluants appropriés comprenant en outre un acide aminé contenant au moins un groupe amino à côté du groupe acide aminé, où ledit acide aminé basique est présent sous forme de base libre.

2. Association pour l'utilisation selon la revendication 1, où ledit acide aminé contenant au moins 1 groupe amino en plus du groupe amino acide, où ledit acide aminé basique est présent sous forme de base libre est choisi parmi la lysine, l'ornithine et l'arginine, de préférence c'est la L-arginine.

3. Association pour l'utilisation selon la revendication 2, où ledit acide aminé contenant au moins un groupe amino en plus du groupe amino acide aminé est la L-arginine.

4. Association pour l'utilisation selon l'une quelconque des revendications 1 à 3, où lesdites compositions orales sous une forme posologique unitaire comprennent entre 50 et 500 mg d'acide nervonique, entre 50 et 500 mg d'extrait de racine séché au curcuma, titré en curcumine.

5. Association pour l'utilisation selon la revendication 3, où la L-arginine sous forme de base libre est présente dans des rapports molaires ≥ 1 par rapport à l'acide nervonique.

6. Association pour l'utilisation selon la revendication 3 ou 5, où la L-arginine est présente dans lesdites compositions orales sous une forme posologique unitaire en des quantités telles que la dispersion aqueuse extemporanée associée a une plage de pH interne comprise entre 7,5 et 8,5.

7. Association pour l'utilisation selon l'une quelconque des revendications 3 à 6, où le rapport pondéral de l'acide nervonique à la L-arginine sous forme de base libre est compris entre 1:1,2 et 1:2,4.

8. Association pour l'utilisation selon l'une quelconque des revendications 1 à 7, où lesdites compositions orales sous une forme posologique unitaire comprennent un complexe de vitamine B et de l'acide folique.

9. Association pour l'utilisation selon la revendication 8, où ledit complexe de vitamine B consiste en au moins l'une des vitamines B1, B2, B3, B5, B6, B12.

10. Association pour l'utilisation selon la revendication 9, où lesdites vitamines BI, B2, B3, B5, B6 sont chacune contenues en des quantités allant de 1 à 50 mg, tandis que chacun desdits B12 et acide folique est contenu en des quantités allant de 1 à 1000 µg.

11. Association pour l'utilisation selon l'une quelconque des revendications 1 à 10, où lesdites compositions orales se présentent sous la forme de comprimés pouvant être avalés et dispersés dans l'eau, de capsules rigides ou molles (gel mou) de gélatine animale ou végétale, de poudres ou de granulés, sous la forme de sachets unidoses, pour une dispersion aqueuse extemporanée.

12. Association pour l'utilisation selon l'une quelconque des revendications 1 à 11, où lesdites compositions orales sous forme posologique unitaire sont des compléments alimentaires.
